# EUROPEAN PATENT APPLICATION

(11) **EP 1 291 085 A2**
(43) Date of publication of application: **12.03.2003**
(21) Application number: 02255924.9
(22) Date of filing: 27.08.2002
(51) Int. Cl.: B01L 7/00, G01N 33/48, G01N 21/47

(54) **Test strip compression element**

(30) Priority: 05.09.2001 US 946690
(71) Applicant: Lifescan, Inc., Milpitas, CA 95035 (US)
(72) Inventor: Olson, Lorin, Scotts Valley, California 95066 (US)
(74) Representative: Mercer, Christopher Paul

(57) **Abstract**

Test strip compression elements for use with test strip assay system and methods for their use are provided. The subject test strip compression elements are configured to create a substantially intimate, evenly distributed contact between a test strip and a heating element of a meter when the test strip is operatively inserted into the meter. In certain embodiments, the subject test strip compression elements may further include one or more protrusions on the contact side of the test strip compression element. In other embodiments, the compression elements may be spring-loaded. Still other embodiments may include a compression element having one or more protrusions, as well as being spring-loaded. Also provided are meters on which the subject test strip compression elements are present, as well as methods for using the same. Kits are also provided that include the subject test strip compression elements for use in practicing the subject methods.

## Description

### INTRODUCTION

### Field Of The Invention

The field of this invention is medical diagnostic devices for measuring the concentration of an analyte in, or a property of, a biological fluid.

### Background Of The Invention

A variety of medical diagnostic procedures involve tests on biological fluids, such as blood, urine, or saliva, and are based on a change in a physical characteristic of such a fluid or an element of the fluid, such as blood serum. The characteristic can be an electrical, magnetic, fluidic, or optical property. When an optical property is monitored, these procedures may make use of a transparent or translucent device to contain the biological fluid and a reagent. A change in light absorption of the fluid can be related to an analyte concentration in, or property of, the fluid.

A growing number of assay formats employ a disposable test strip, a fluidic device or a card which is used in conjunction with a meter. The disposable fluid device receives the sample to be assayed and includes any reagents necessary for the assay to be conducted. The test strip also typically includes one or more flow paths through which the sample flows during the assay.

As mentioned above, these test strips are typically used in conjunction with a meter which is capable of receiving a signal from a measurement area of the card. To receive the signal from the measurement area, the test strip is generally inserted into an opening in the meter so that at least the measurement area of the test strip is present inside the meter. Examples of assay systems that are made up of these types of disposable test strips and meters may be found in EP-A-0 974 840 and US Application Serial No. 09/356248, filed July 16, 1999.

Many of these assays require that the sample be maintained as a certain temperature, for example at or near body temperature (about 37°C), in order for the meters to obtain accurate measurements. Accordingly, the meters into which the test strips are inserted typically include one or more heating elements used to heat and maintain the sample on the test strip at or substantially near an optimum temperature.

Another related factor in the ability to obtain accurate test results is the ability to uniformly and reproducibly heat the measurement area each time, i.e., the ability to heat each measurement area at substantially the same temperature each time. Where more than one measurement area is included on a test strip, e.g., one containing sample, and one or more containing a control solution, it is similarly important to be able to heat each measurement area similarly, i.e., heat each measurement area at substantially the same temperature as any other measurement area on the strip. It will be apparent that differential heating of measurement areas may cause erroneous results. For example, if a control measurement area is heated properly, but the sample measurement area is not, the results relating to the sample may be inaccurate.

In conventional meters, the test strips are held down in the meter with two contact points on the periphery of the meter, where such contact points are positioned at or near the periphery of the test strip, i.e., on opposing sides of a measurement area of the test strip. One disadvantage with using this method used to hold down and uniformly heat the test strip is that uniform heating is dependant upon the flatness of the test strip. If the strip is not flat, then it will not be heated uniformly. A slight air gap between the test strip and the heater could significantly compromise heating. As such, reproducibility may be problematic for the above-described reason. Furthermore, such methods are inefficient in that the entire strip is heated, as opposed to just the measurement area.

As such, there is a need for the development of a device that is capable of providing uniform and reproducible heating of the sample measurement area(s) of a test strip so that accurate measurements may be made.

### Summary Of The Invention

Test strip compression elements for use with test strip assay system and methods for their use are provided. The subject test strip compression elements are configured to create a substantially intimate, evenly distributed contact between a test strip and a heating element of a meter when the test strip is operatively inserted into the meter. In certain embodiments, the subject test strip compression elements may further include one or more protrusions on the contact side of the test strip compression element. In other embodiments, the compression elements may be spring-loaded. Still other embodiments may include a compression element having one or more protrusions, as well as being spring-loaded. Also provided are meters on which the subject test strip compression elements are present, as well as methods for using the same. Kits are also provided that include the subject test strip compression elements for use in practicing the subject methods.

### Brief Descriptions Of The Drawings

Figure 1A shows a top view of an exemplary embodiment of the subject test strip compression elements.

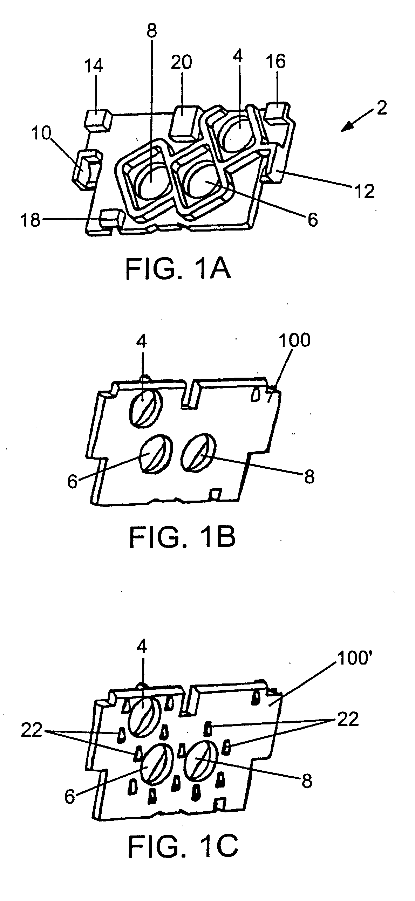

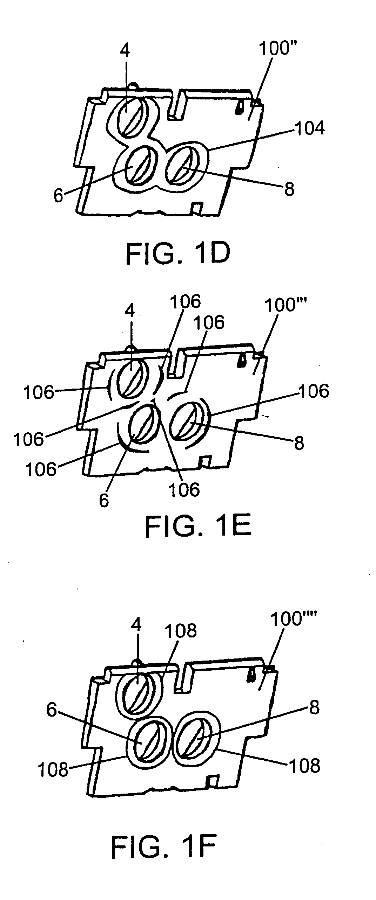

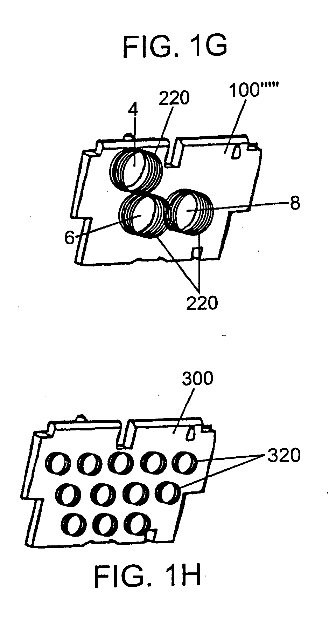

Figures 1B-1F show exemplary embodiments of the test strip contact side of a test strip compression element. Figure 1B shows an exemplary embodiment of a contact side having substantially planar surface. Figure 1C shows an exemplary embodiment of a contact side having a plurality of protrusions. Figure 1D shows an exemplary embodiment of a contact side having a single continuous protrusion. Figure 1E shows an exemplary embodiment of a contact side having a plurality of different shaped and sized protrusions. Figure 1F shows an exemplary embodiment of a contact side having protrusions which substantially or completely encircle the apertures of the compression element. Figure 1G shows an exemplary embodiment of a contact side having protrusions which are coils, where such coils substantially or completely encircle the apertures of the compression element. Figure 1H shows an exemplary embodiment of a contact side without apertures and having a plurality of coils.

Figure 2A shows an overhead view of a meter device with the test strip compression elements of Figures 1A and 1B and a test strip positioned therein. Figures 2B and 2C show top and bottom views of the proximal portion of the meter of Figure 2A.

Figure 3 provides a plan view of an exemplary test strip suitable for use with the subject invention.

Figure 4 provides an exploded view of the test strip of Figure 3.

Figure 5 provides a perspective view of the test strips of Figures 4 and 5.

Figure 6 shows an exemplary embodiment of the described test strip of Figures 3, 4 and 5 having a plurality of measurement areas.

Figure 7 shows an exemplary embodiment of a meter suitable for use with the subject invention. Figure 7a shows an alternative embodiment of an element of the meter of Figure 7.

Figure 8 is a graph of data used to determine PT time.

### Detailed Description Of The Invention

Test strip compression elements for use with test strip assay system and methods for their use are provided. The subject test strip compression elements are configured to create a substantially intimate, evenly distributed contact between a test strip and a heating element of a meter when the test strip is operatively inserted into the meter. In certain embodiments, the subject test strip compression elements may further include one or more protrusions on the contact side of the test strip compression element. In other embodiments, the compression elements may be spring-loaded. Still other embodiments may include a compression element having one or more protrusions, as well as being spring-loaded. Also provided are meters on which the subject test strip compression elements are present, as well as methods for using the same. Kits are also provided that include the subject test strip compression elements for use in practicing the subject methods.

Before the present invention is described, it is to be understood that this invention is not limited to the particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. Thus, for example, reference to "a heating element" includes a plurality of such heating elements and reference to "the meter" includes reference to one or more meters and equivalents thereof known to those skilled in the art, and so forth.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEVICES

As summarized above, the present invention provides test strip compression elements suitable for use with test strip meters. The subject test strip compression elements are characterized by being configured to create a substantially intimate, evenly distributed contact between the test strip and a heating element of the meter when the test strip is operatively inserted into the meter. In further describing the subject invention, the subject test strip compression elements will be described first, followed by a description of test strips and meters suitable for use with the subject test strip compression elements.

### Test Strip Compression Elements

The subject test strip compression elements may be of any convenient shape, where the shape of the subject test strip compression elements may depend on a variety of factors such as the particular configuration of the meter and/or test strip used with the subject test strip compression elements. For example, the test strip compression elements may be of a regular shape such as a substantially square, rectangle, triangular, circular, oval, elliptical shape and the like or may be an irregular shape. The material from which the subject test strip compression elements are made may vary depending on a variety of factors such as the particular meter used, etc., where suitable materials include plastics, metals, and the like. The test strip compression elements may be fabricated using any convenient protocol, where representative protocols include machining, injection molding, compression molding, casting and the like.

Similarly, the size of the test strip compression elements may vary, where such size may depend on a variety of factors such as the particular configuration of the meter and/or test strip used with the subject test strip compression elements. In those embodiments having a substantially rectangular shape, the length of the element is typically about 4 to 50 mm, usually about 10 to 40 mm and more usually about 20 to 30 mm, the width is typically about 2 to 40 mm, usually about 8 to 25 mm and more usually about 10 to 20 mm and the thickness is typically about 0.5 to 10 mm, usually about 1 to 6 mm and more usually about 2 to 3 mm.

As noted above, the subject invention may be used with a variety of assays. In certain assays, it may be important that light can be transmitted through the measurement area of the test strip. Accordingly, the test strip compression elements may also include one or more apertures, where such apertures are configured to substantially align with the measurement area(s) of the test strip so that the measurement area can be illuminated by a light source on one side and the transmitted light measured on the opposite side. However, it will be apparent to one of skill in the art that the apertures will typically be absent in the case of reflective optics, i.e., apertures will not usually be present if reflectance is measured.

When present, the number of apertures will vary depending on the particular test strip being used, however, typically the number will range from about 1 to 25, usually from about 1 to 10 and more usually from about 1 to 5. It will be apparent that the size and/or shape of the apertures may vary depending on the particular test strip used. In other words, the size and shape of the apertures will substantially correspond to the size and/or shape of the measurement area over which it is positioned. Accordingly, the shape of any one aperture be of a regular shape such as a substantially square, rectangle, triangular, circular, oval, elliptical shape and the like or may be or an irregular shape. In those embodiments having a substantially circular shape, the diameter of the circular aperture will typically range from about 0.5 to 20 mm, usually from about 2 to 10 mm and more usually from about 3 to 5 mm.

In certain embodiments of the subject devices, the subject test strip compression elements are further characterized by having at least one protrusion, oftentimes a plurality of such protrusions. More specifically, the at least one protrusion is positioned on a surface (i.e., the contact surface) of the element, i.e., the surface or side of the compression elements that is associated with, or directly opposed to, the surface of a test strip when the test strip is inserted into a meter, where such protrusions are configured to directly contact the test strip beneath the elements, typically in the region of the test strip in close proximity to, or in the immediate area of, a measurement area. In other words, at least one protrusion, sometimes a plurality of such protrusions, is usually positioned adjacent an aperture, as described above, such that when a test strip is inserted into a meter, the at least one protrusion, or a plurality of protrusions, is in direct opposition to, and thus positioned adjacent the periphery of a measurement area of the test strip, i.e., in contact with the area immediately close or near to a measurement area. Where a plurality of apertures are present, each aperture will usually be associated with at least one protrusion, usually each will be associated with a plurality of protrusions, where such protrusions may be the same or different from protrusions associated with another aperture. In other words, the apertures may "share" protrusions. Protrusions may take a variety of forms, including, but not limited to coils, solid protrusions and the like. The protrusions may be made of any convenient material and will often be the same material as the compression element, i.e., plastic, metal, and the like and will typically be one unitary piece of construction. However, in certain embodiments, the at least one protrusion may be of a different material such as an elastomeric material, i.e., rubber, which may also include a coating to facilitate test strip insertion into the meter, such as a Teflon® coating or the like.

In those embodiments having protrusions associated with the apertures, typically each aperture will have from about 1 to 15 protrusions associated with it, usually from about 1 to 10 and more usually from about 1 to 6 or 1 to 5, where the number may vary depending on the size and/or shape of the protrusions, etc. The at least one protrusion may have any convenient shape, as long as they are shaped to perform their particular functions, as described. Similarly, the size of the at least one protrusion may vary, depending on a number of factors such as the number or protrusions present, etc. By way of example and not limitation, in those embodiments having about 4 to 6 protrusions adjacent each aperture, where the protrusions have a substantially rectangular shape, the length of each protrusion ranges from about 0.1 to 6 mm, usually from about 0.5 to 3 mm and more usually from about 1 to 1.5 mm, the width of each protrusion ranges from about 0.1 to 3 mm, usually from about 0.3 to 1 mm and more usually from about 0.4 to 0.6 mm and the thickness of each protrusion ranges from about 0.1 to 2 mm, usually from about 0.2 to 0.8 mm and more usually from about 0.3 to 0.5 mm. It is important to note that a test strip compression elements may have a combination of different protrusions, for example may include protrusions of different or various sizes and shapes.

As mentioned above, the at least one protrusion is typically associated with or positioned adjacent an aperture of the compression elements, e.g., substantially around the perimeter of such an aperture. In other words, the at least one protrusion is configured to be positioned substantially adjacent to, and in contact with, a measurement area of a test strip when the test strip is inserted into a meter, e.g., substantially around the perimeter of a measurement area. For example, typically, from about 1 to 15 protrusions are associated with each measurement area of the test strip, usually from about 1 to 10 protrusions are associated with each measurement area of the test strip and more usually from about 1 to 6 or 1 to 5 protrusions are associated with each measurement area of the test strip, where the number may vary depending on the size of the bumps, etc. Accordingly, each protrusion is typically positioned a distance from about 0 to 3 mm from an aperture, usually from about 0 to 1 mm and more usually from about 0.4 to 0.6 mm from an aperture. Of course, this distance may vary depending on the particular test strip configuration used. It will be obvious that in those embodiments having no apertures but having protrusions, the at least one protrusion will similarly be configured and positioned on the test strip compression element to be similarly associated with the measurement area(s) of a test strip (adjacent to or substantially around a measurement area of a test strip when the test strip is inserted into a meter, e.g., around or adjacent the perimeter of a measurement area, as described above).

In certain embodiments of the subject invention, the test strip compression elements are spring loaded. That is, the test strip compression elements are configured to move in a first or un-biased direction and a second or biased direction, where the compression elements are biased such that it is positioned to be in direct contact with a test strip when the test strip is inserted into a meter. Accordingly, the test strip compression elements may be associated with a spring element. For example, the compression elements may include an attachment or alignment means, e.g., a groove, channel or the like, configured to receive one or more corresponding spring elements such as one or more spring leaves associated with a meter located near the test strip receiving area of the meter. As such, when engaged with the corresponding spring element, the test strip compression element is configured to be biased or forced against a test strip that has been inserted into the meter. In those embodiments having at least one protrusion on the test strip compression element, the at least one protrusion is configured to be biased towards the test strip inserted into the meter such that the at least one protrusion contacts the test strip. In other words, the test strip compression element, or the at least one protrusion of the test strip compression elements, is biased so as to contact and exert a force upon the test strip, due to the spring loaded character of the compression element, and more particularly exert a force upon the one or more areas associated with the one or more measurement areas of the test strip.

Accordingly, the subject test strip compression elements, and/or the one or more protrusions of the test strip compression elements, with or without the assistance of a spring-loaded force, are configured to apply a force upon a test strip sufficient to produce a substantially intimate, evenly distributed contact between a heating element of the meter into which the test strip is inserted and one or more of (1) the test strip, (2) one or more measurement areas of the test strip or (3) the test strip and one or more measurement areas thereof. Accordingly, the force applied by the compression elements press down upon a test strip inserted into the meter and/or the one or more measurement areas thereof, with enough force to cause the test strip and/or the one or more measurement areas thereof, to make substantially intimate, evenly distributed contact with the heating element of the meter. By substantially intimate contact it is meant that the test strip, and/or the one or more measurement areas thereof, forms a good or substantially thermal conducting contact with the heating element, i.e., the one or more measurement areas touch or lie directly on or substantially adjacent the heating element of the meter, where typically the measurement areas are in direct contact with the heating element. Of course, only that force necessary to contact the test strip, and/or the one or more measurement areas thereof, with the heating element is used. That is, only force that will not cause adverse consequences to the test strip or the meter, and the heating element of the meter particularly, or otherwise result in erroneous test results, will be used. Typically, the force applied by the compression elements and/or the at least one protrusion of the compression elements ranges from about 0.02 to 3 lbs. more usually from about 0.1 to 2.5 lbs. and more usually from about 0.2 to 1 lb.

As described, the subject invention is configured such that the force applied to the test strip, or a portion thereof, will create a substantially intimate, evenly distributed contact between the test strip and/or the one or more measurement areas thereof, and a heating element of a meter into which the test strip is inserted. In certain embodiments, the force will be distributed substantially uniformly or evenly over the surface of the test strip in contact with the subject invention so that each protrusion will exert the same amount of force upon a test strip inserted into the meter and each will conform to the test strip. In other words, every portion of the test strip compression elements may be biased toward the test strip with the same force as every other portion of the test strip compression elements.

Regardless of whether the force is substantially evenly distributed over the surface area of the test strip compression element or not, the compression element and/or the at least one protrusion of the compression element, will exert a force upon the test strip and/or each measurement area of a test strip inserted into a meter such that the test strip and/or each measurement area thereof is evenly contacted, or has a substantially evenly distributed contact, with the heating element, i.e., the test strip and/or measurement area thereof, is positioned a distance (where such distance may be zero where the measurement areas directly contact the heating element) from the heating element of the meter that is substantially the same distance as every other measurement area so that the measurement areas all receive substantially the same amount of heat from the heating element. For example, in those test strip embodiments having three measurement areas, the compression element, and/or protrusions thereof, is configured to press down to conform to each measurement area of the test strip and force each measurement area to contact the heating element, where each measurement area contacts or is associated with the heating element in substantially the same way, (distance, orientation, etc.) as the other measurement areas.

The subject test strip compression elements will now be further described in reference to the Figures, where like numerals represent like elements or features. Figure 1A shows a top view of an exemplary embodiment of the subject test strip compression elements, i.e., a view of the top or non-contact side, i.e., the side that does not contact a test strip when a test strip is inserted into a meter, of test strip compression elements 2. Compression element 2 includes three apertures 4, 6 and 8, where each aperture is configured to be positioned substantially over a respective measurement area of a test strip. Test strip compression element 2 also includes grooves 10 and 12 which are configured to receive respective, corresponding spring elements such as spring leaves associated with a meter so that the compression element can have spring-loaded character, i.e., has a floating-like characteristic. However, in certain embodiments as described above, the subject compression elements may not be spring-loaded. In this particular embodiment, test strip compression element 2 also includes additional attachment elements 14, 16, 18 and 20 which are configured to securely capture or attach the test strip compression element 2 to a meter, where any number ranging from about 0 to about 50 attachment elements may be present. In this particular embodiment, test strip compression element 2 is manufactured as a separate component or piece with respect to a meter; however, it will be apparent that the test strip compression elements 2 and a meter may be manufactured as one piece or may be a unitary piece of construction.

Figure 1B shows a view of an exemplary embodiment of the under side or contact side of a test strip compression element, such as test strip compression element 2 of Figure 1A. In this particular embodiment, the contact side 100, i.e., the side that contacts a test strip when a test strip is inserted into a meter, is substantially planar, i.e., does not include any protrusions.

Figure 1C shows a view of another exemplary embodiment of the under side or contact side of a test strip compression element, such as test strip compression element 2 of Figure 1A. The second or contact side 100' includes a plurality of protrusions 22. The plurality of protrusions is positioned adjacent each aperture of the compression element, particularly adjacent the perimeter of each aperture, i.e., positioned so that when a test strip is inserted into a meter, the plurality of protrusions is associated and in contact with the measurement areas of the test strip. The plurality of protrusions 22 is configured and positioned to contact a test strip and exert a force thereupon such that the test strip and/or measurement areas thereof are substantially intimately and evenly contacted with a heating element of a meter into which the test strip is inserted.

Figure 1D shows a view of another exemplary embodiment of the under side or contact side of a test strip compression element, such as test strip compression element 2 of Figure 1A. In this particular embodiment, contact side 100" includes protrusion 104, where protrusion 104 is a single, continuous protrusion that is adjacent the apertures 4, 6 and 8. The protrusion 104 is configured and positioned to contact a test strip and exert a force such thereupon such that the test strip and/or measurement areas thereof are substantially intimately and evenly contacted with a heating element of a meter into which the test strip is inserted.

Figure 1E shows a view of yet another exemplary embodiment of the under side or contact side of a test strip compression element, such as test strip compression element 2 of Figure 1A. In this particular embodiment, contact side 100"' includes a plurality of protrusions 106, where such protrusions may of the same or different sizes and/or shapes, herein shown in this embodiment as different sizes and shapes. The plurality of protrusions 106 is configured and positioned to contact a test strip and exert a force such thereupon such that the test strip and/or measurement areas thereof are substantially intimately and evenly contacted with a heating element of a meter into which the test strip is inserted.

Figure 1F shows a view of yet another exemplary embodiment of the under side or contact side of a test strip compression element, such as test strip compression element 2 of Figure 1A. In this particular embodiment, contact side 100'''' includes a plurality of protrusions 108, where each protrusion substantially, if not completely, surrounds or encircles an aperture of the compression element. The plurality of protrusions 108 is configured and positioned to contact a test strip and exert a force such thereupon such that the test strip and/or measurement areas thereof are substantially intimately and evenly contacted with a heating element of a meter into which the test strip is inserted.

Figure 1G shows yet another exemplary embodiment of the under side or contact side of a test strip compression element, such as test strip compression element 2 of Figure 1A. In this particular embodiment, contact side 100""' includes a plurality of coils 220, where each coil substantially, if not completely, surrounds or encircles an aperture of the compression element. The plurality of coils 220 is configured and positioned to contact a test strip and exert a force such thereupon such that the test strip and/or measurement areas thereof are substantially intimately and evenly contacted with a heating element of a meter into which the test strip is inserted.

Figure 1H shows yet another exemplary embodiment of the under side or contact side of a test strip compression element, such as test strip compression element 2 of Figure 1A; however, in this particular embodiment, the compression element does not include apertures. In other words, the top or non-contact side of this compression element is substantially the same as the top side of the compression element of Figure 1A, except it does not have apertures and the contact side 300 includes a plurality of coils 320. The plurality of coils 320 is configured and positioned to contact a test strip and exert a force such thereupon such that the test strip and/or measurement areas thereof are substantially intimately and evenly contacted with a heating element of a meter into which the test strip is inserted. It will be understood that any of the above mentioned embodiments, i.e., embodiments describing protrusions, are applicable to compression elements having no apertures. In other words, any of the protrusions described above may also be present on compression elements having no apertures.

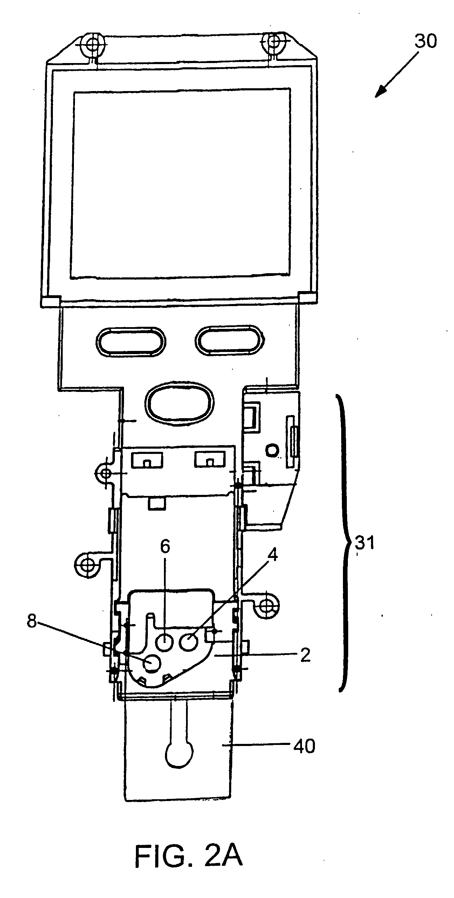

Figure 2A shows an overhead view of a meter device with a test strip compression element 2 of Figures 1A to 1H (herein shown with apertures). Also shown in Figure 2A is test strip 40 positioned in meter 30 such that apertures 4, 6, and 8 are positioned over the test strip's three measurement areas. Meters and test strips suitable for use with the subject invention will be described in greater detail below.

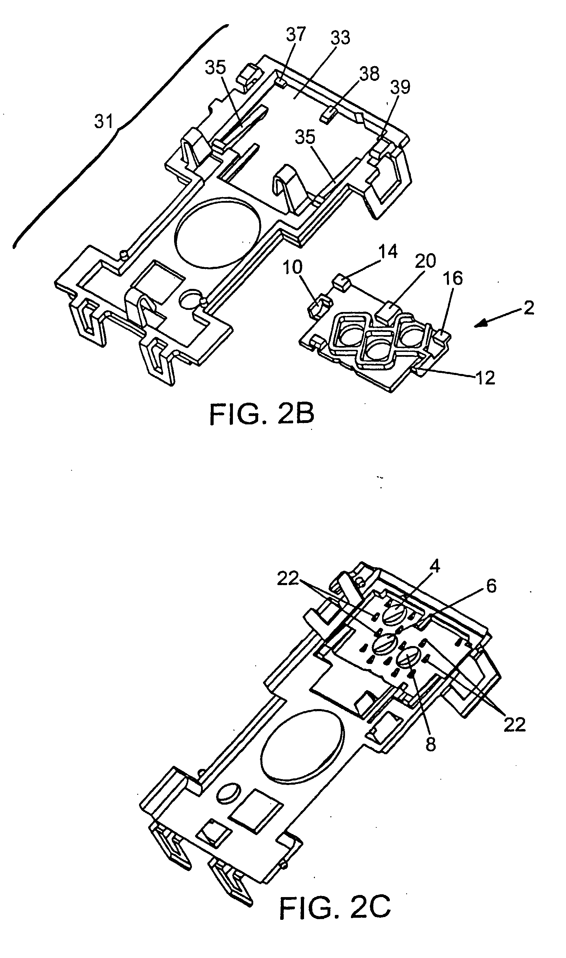

Figures 2B and 2C show top and bottom views of the proximal portion 31 of meter 30, i.e., the portion of the meter that includes a test strip compression element. Figure 2B shows proximal portion 31 and corresponding test strip compression element 2 of Figures 1A to 1G. Proximal portion 31 includes a region 33 for receiving a test strip compression element 2. As such, it includes leaf springs 35 to secure the test strip compression element 2 thereto and enable the test strip compression element associated therewith to be spring loaded; particularly, leaf springs 35 are configured to receive corresponding attachment elements 10 and 12 of the compression element 2. Proximal portion 31 also includes additional securing elements such as securing elements 37, 38 and 39, which are configured to associate with attachment points 14, 20 and 16 of the compression element 2, respectively. Figure 2C shows a of the under side of proximal portion 31 having test strip compression element 2 attached.

### SYSTEMS

The above described test strip compression elements find use with systems that include fluidic devices or test strips and meters, as described below.

### Test Strips

The fluidic test strips of the systems with which the subject strip compression elements find use are fluidic devices that generally include a sample application area; a bladder, to create a suction force to draw the sample into the device; a measurement area, in which the sample may undergo a change in an optical parameter, such as light scattering; and a stop junction to precisely stop flow after filling the measurement area. In many embodiments, the devices are substantially transparent over the measurement area, so that the area can be illuminated by a light source on one side and the transmitted light measured on the opposite side.

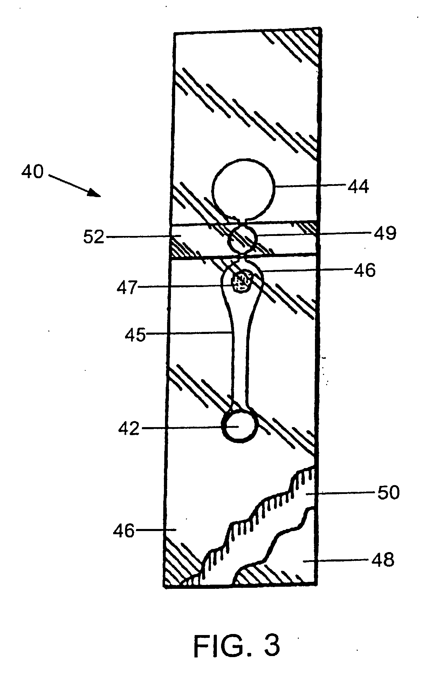

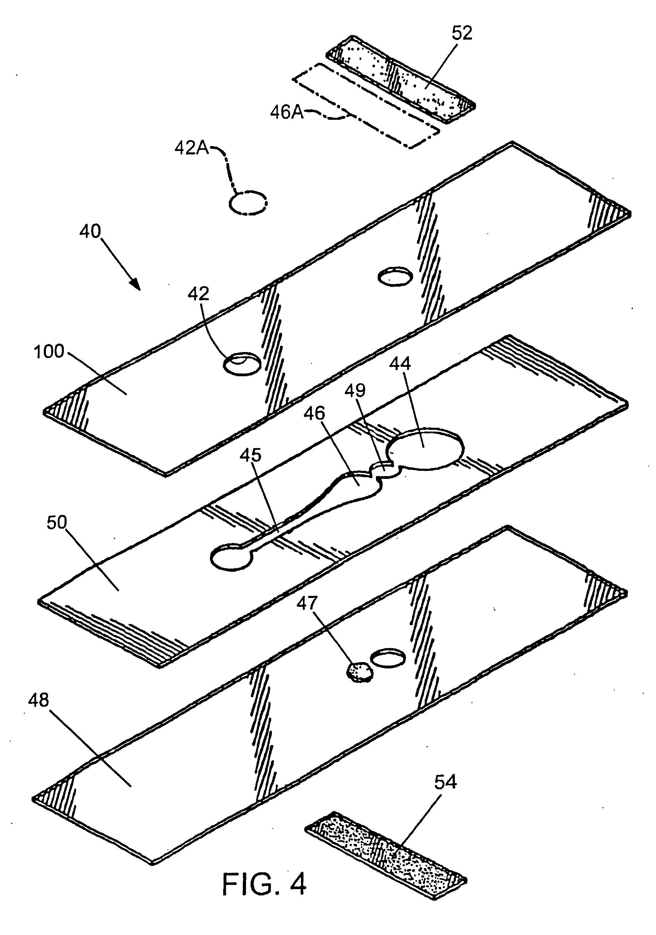

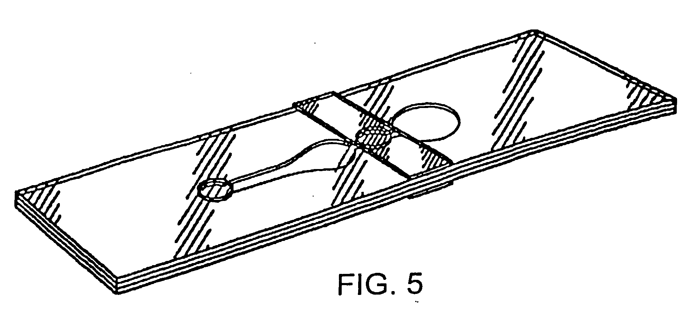

An exemplary test strip with which the subject compression elements find use is shown in Figs. 3, 4 and 5. Fig. 3 provides a plan view of a test strip 40, while Fig. 4 provides an exploded view and Fig. 5 provides a perspective view of the same representative test strip. Sample is applied to sample port 42 after bladder 44 has been compressed. Clearly, the region of layer 46 and/or layer 48 that adjoins the cutout for bladder 44 must be resilient, to permit bladder 44 to be compressed. Polyester of about 0.1 mm thickness has suitable resilience and springiness. Typically, top layer 46 has a thickness of about 0.125 mm, bottom layer 48 about 0.100 mm. When the bladder is released, suction draws sample through channel 45 to measurement area 46, which typically contains a reagent 47. In order to ensure that measurement area 46 can be filled with sample, the volume of bladder 44 is usually at least about equal to the combined volume of channel 45 and measurement area 46. If measurement area 46 is to be illuminated from below, layer 48 must be transparent where it adjoins measurement area 46.

As shown in Figs. 3, 4, and 5, stop junction 49 adjoins bladder 44 and measurement area 46; however, a continuation of channel 45 may be on either or both sides of stop junction 49, separating the stop junction from measurement area 46 and/or bladder 44. When the sample reaches stop junction 49, sample flow stops. The principle of operation of stop junctions is described in U.S. Patent 5,230,866.

As shown in Fig. 4, all the above elements are formed by cutouts in intermediate layer 50, sandwiched between top layer 100 and bottom layer 48. Preferably, layer 50 is double-sided adhesive tape. Stop junction 49 is formed by an additional cutout in layer 100 and/or 48, aligned with the cutout in layer 50 and sealed with sealing layer 52 and/or 54. Typically, as shown, the stop junction comprises cutouts in both layers 100 and 48, with sealing layers 52 and 54. Each cutout for stop junction 49 is at least as wide as channel 45. Also shown in Fig. 4 is an optional filter 42A to cover sample port 42. The filter may separate out red blood cells from a whole blood sample and/or may contain a reagent to interact with the blood to provide additional information. A suitable filter comprises an anisotropic membrane, preferably a polysulfone membrane of the type available from Spectral Diagnostics, Inc., Toronto, Canada. Optional reflector 46A may be on, or adjacent to, a surface of layer 100 and positioned over measurement area 46. If the reflector is present, the device becomes a transflectance device.

The test strip pictured in Fig. 4 and described above is typically formed by laminating thermoplastic sheets 100 and 48 to a thermoplastic intermediate layer 50 that has adhesive on both of its surfaces. The cutouts that form the elements shown in Figure 3 may be formed, for example, by laser- or die-cutting of layers 100, 48, and 50. Alternatively, the device can be formed of molded plastic. Typically, the surface of sheet 48 is hydrophilic. (Film 9962, available from 3M, St. Paul, MN.) However, the surfaces do not need to be hydrophilic, because the sample fluid will fill the device without capillary forces. Thus, sheets 100 and 48 may be untreated polyester or other thermoplastic sheet, well known in the art. Similarly, since gravity is not involved in filling, the device can be used in any orientation. Unlike capillary fill devices that have vent holes through which sample could leak, these types of devices vent through the sample port before sample is applied, which elements that the part of the strip that is first inserted into the meter is without an opening, reducing the risk of contamination.

Other test strip configurations are also possible, where such alternative device configurations include those that have: a bypass channel; multiple parallel measurement areas; and or multiple in series measurement areas, etc. In addition, the above described laminated structures can be adapted to injection molded structures. A variety of alternative fluidic devices are described in EP-A-0 974 840 and US Application Serial No. 09/356248, filed July 16, 1999.

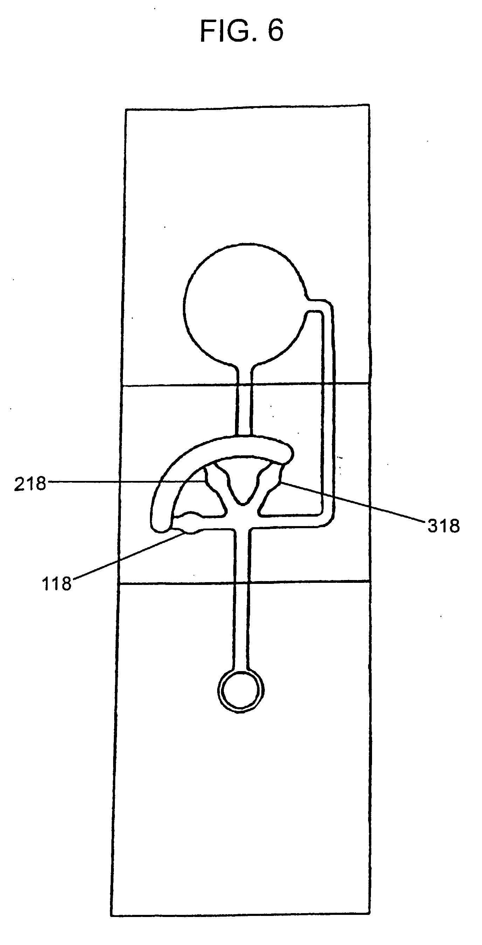

Figure 6 shows an exemplary embodiment of the above described test strip having a plurality of measurement areas. Figure 6 shows a test strip that includes parallel measurement areas 118, 218 and 318. As described above, one or more of the measurement areas may include a control. For example, measurement area 118 may contain thromboplastin and measurement areas 218 and 318 may contain controls. For example, measurement area 218 may contain thromboplastin, bovine eluate and recombinant Factor VIIa and measurement area 318 may contain thromboplastin and bovine eluate alone. Thus, three measurements may be made on this test strip.

### Meters

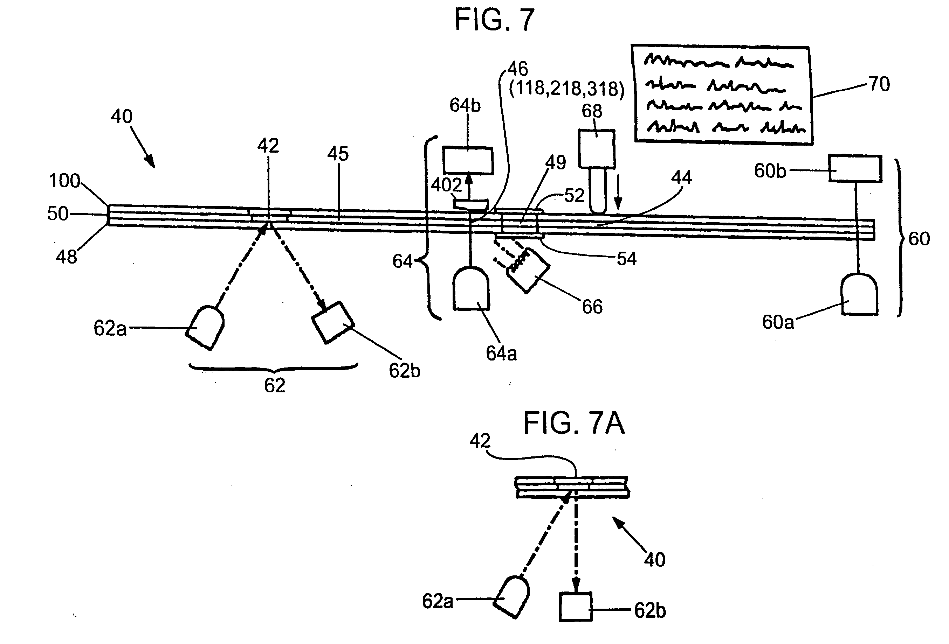

The subject test strip compression elements find use with meters, generally automated meters, that are designed for use with the above described test strip compression elements. As noted above, the meter and test strip compression elements may be fabricated as one unit, i.e., a unitary piece of construction or may be separate units or components. An exemplary meter is depicted in Figure 7, wherein a representative test strip 40 is inserted into the meter. The meter shown in Figure 7 includes strip detector 60 (made up of LED 60a and detector 60b), sample detector 62 (made up of light source 62a and detector 62b), measurement system 64 (made up of LED 64a and detector 64b) a test strip compression element 402, for example any of the above described compression elements such as those described in Figures 1A to 1G, and heater 66. The device further includes a bladder actuator 68. The bladder actuator is, in many embodiments, actuated by the strip detector 60 and the sample detector 62, such that when a strip is inserted into the meter and detected by the strip detector, the bladder actuator is depressed, and when the sample is added to the fluidic device or strip inserted into the meter, the bladder actuator is withdrawn so as to decompress the bladder and concomitantly pull sample into the measurement area of the device via the resultant negative pressure conditions. Also present is a meter display 70 that provides for an interface with the user.

### METHODS OF USE

The above described systems and devices that include the subject test strip compression elements are suitable for use in a variety of analytical tests of biological fluids, such as determining biochemical or hematological characteristics, or measuring the concentration in such fluids of analytes such as proteins, hormones, carbohydrates, lipids, drugs, toxins, gases, electrolytes, etc. The procedures for performing these tests have been described in the literature. Among the tests, and where they are described, are the following: (1) Chromogenic Factor XIIa Assay (and other clotting factors as well): Rand, M.D. et al., Blood, 88, 3432 (1996); (2) Factor X Assay: Bick, R.L. Disorders of Thrombosis and Hemostasis: Clinical and Laboratory Practice. Chicago, ASCP Press, 1992.; (3) DRVVT (Dilute Russells Viper Venom Test): Exner, T. et al., Blood Coag. Fibrinol., 1, 259 (1990); (4) Immunonephelometric and Immunoturbidimetric Assays for Proteins: Whicher, J.T., CRC Crit. Rev. Clin Lab Sci. 18:213 (1983); (5) TPA Assay: Mann, K.G., et al., Blood, 76, 755, (1990).; and Hartshorn, J.N. et al., Blood, 78, 833 (1991); (6) APTT (Activated Partial Thromboplastin Time Assay): Proctor, R.R. and Rapaport, S.I. Amer. J. Clin. Path, 36, 212 (1961); Brandt, J.T. and Triplett, D.A. Amer. J. Clin. Path., 76, 530 (1981); and Kelsey, P.R. Thromb. Haemost. 52, 172 (1984); (7)HbAlc Assay (Glycosylated Hemoglobin Assay): Nicol, D.J. et al., Clin. Chem. 29, 1694 (1983); (8) Total Hemoglobin: Schneck et al., Clinical Chem., 32/33, 526 (1986); and U.S. Patent 4,088,448; (9) Factor Xa: Vinazzer, H., Proc. Symp. Dtsch. Ges. Klin. Chem., 203 (1977), ed. By Witt, I; (10) Colorimetric Assay for Nitric Oxide: Schmidt, H.H., et al., Biochemica, 2, 22 (1995).

The above described systems and devices are particularly well suited for measuring blood-clotting time - "prothrombin time" or "PT time, " as more fully described in EP-A-0 974 840 and US Application Serial No. 09/356248, filed July 16, 1999. The modifications needed to adapt the device for applications such as those listed above require no more than routine experimentation.

In the subject methods, a substantially intimate, evenly distributed contact between a heating element and one or more of (1) the test strip, (2) one or more measurement areas of the test strip, or (3) the test strip and one or more measurement areas of the test strip, is created, in certain embodiments by a force exerted thereto. As mentioned above, by substantially intimate contact it is meant that the test strip, and/or the one or more measurement areas thereof, forms a good or substantially thermally conductive contact with the heating element, i.e., the one or more measurement areas touch or lie directly on or substantially adjacent the heating element of the meter, where typically the measurement areas are in direct contact with the heating element. Also as mentioned above, by substantially evenly distributed is meant the exertion of a force upon the test strip and/or each measurement area of a test strip inserted into a meter such that the test strip and/or each measurement area thereof is evenly contacted, or has a substantially evenly distributed contact, with the heating element, i.e., the test strip and/or measurement area thereof, is positioned a distance (where such distance may be zero where the measurement areas directly contact the heating element) from the heating element(s) of the meter that is substantially the same distance as every other measurement area so that the measurement areas all receive substantially the same amount of heat from the heating element. For example, in those test strip embodiments having three measurement areas, the compression element, and/or protrusions thereof, is configured to press down to conform to each measurement area of the test strip and force each measurement area to contact the heating element, where each measurement area contacts or is associated with the heating element in substantially the same way, (distance, orientation, etc.) as the other measurement areas.

Accordingly, a test strip is inserted into a meter. Once inserted, a pressure or force is exerted onto the test strip to create a substantially intimate, evenly distributed contact between the test strip and/or measurement area(s) of the test strip and the heater. In the subject methods, the force is exerted by a subject compression element, at least by way of the weight of the compression element. Such compression forces may further include a spring loaded compression means and/or one or more protrusions on the contact side of the compression element. The subject method will now be described in reference to the subject devices.

In using the above systems that include the subject test strip compression element, the first step the user performs is to turn on the meter, where such a meter includes a subject test strip compression element, thereby energizing the strip detector, sample detector, measurement system and heater of the meter. The second step is to insert a test strip into the meter, where the strip is inserted between the test strip compression element and a meter surface. In certain embodiments, e.g., those embodiments having a spring loaded test strip compression element, the one or more spring leaflets are displaced by the insertion of the test strip, i.e., the test strip urges the spring leaflets to move slightly upward to an un-biased position, to accommodate insertion of the test strip into the meter. Once the test strip is positioned between the meter and the compression element, the leaflets are then biased back towards the strip, i.e., moved to a second position, to exert a force on the test strip, where such a force creates a substantially intimate, uniformly distributed contact between the test strip, and/or the measurement areas of the test strip, and the heater.

In other words, in certain embodiments of the subject methods, the compression element, and/or protrusions thereof, exerts a force on the test strip and/or measurement area(s) of the test strip. Thus, in those embodiments of the subject devices having at least one protrusion, the strip is inserted such that the one or more measurement areas of the test strip are associated with the one or more protrusions of the subject test strip compression elements. In many embodiments, each measurement area is aligned with an aperture, if present, of the compression area such that at least one protrusion associated with the aperture is, in turn, associated with a measurement area and the at least one protrusion applies or exerts a force to the area around or immediately adjacent to the measurement area, where such applied force result in substantially intimate contact between each measurement area and the heating element of the meter. (Of course, in those embodiments where no apertures are present, the measurement areas of the test strip are similarly aligned with at least one protrusion.)

Only that force necessary to create a substantially intimate, evenly distributed contact between the test strip and the heater of the meter is applied. That is, only force that will not cause adverse consequences to the strip or the meter, and the heating element of the meter particularly, or otherwise cause erroneous test results, will be applied.

A feature of the subject methods is that the force applied to the test strip is reproducible. By reproducible is meant that substantially the same force will be applied to every test strip associated with the test strip compression element. Typically, the force applied by the compression element, with or without protrusions and/or a spring loaded force, ranges from about 0.02 to 3 lbs. more usually from about 0.1 to 2.5 lbs. and more usually from about 0.2 to 1 lb.

Usually, the strip is not transparent over at least a part of its area, so that an inserted strip will block the illumination by LED 60a of detector 60b. (More usually, the intermediate layer is formed of a non-transparent material, so that background light does not enter measurement system 64.) Detector 60b thereby senses that a strip has been inserted and triggers bladder actuator 68 to compress bladder 44. A meter display 70 then directs the user to apply a sample to sample port 42 as the third and last step the user must perform to initiate the measurement sequence. The empty sample port is reflective. When a sample is introduced into the sample port, it absorbs light from LED 62a and thereby reduces the light that is reflected to detector 62b. That reduction in light, in turn, signals bladder actuator 68 to release bladder 44. The resultant suction in channel 45 draws sample through measurement areas such as measurement areas 118, 218 and 318 of Figure 6 or measurement area 46 of Figures 1A and 1B to stop junction 49. The sample drawn into the measurement areas is heated by heating element 66. Typically, sample temperature is maintained at about 37°C by heater 66. More specifically, the measurement areas are pressed against heater element 66 substantially evenly such that, as described above, each measurement area receives or observes the same amount of heat as any other measurement area and that measurement areas of different tests trips inserted into the meter each receive or observe the same force by the test strip compression elements and thus the same amount of heat each time. The light from LED 64a passes through measurement areas 118, 218 and 318, and detector 64b monitors the light transmitted through the sample as it is clotting. Analysis of the transmitted light as a function of time (as described below) permits a calculation of the PT time, which is displayed on the meter display 70.

As described above, the detector senses a sample in sample port 42, simply by detecting a reduction in (specular) reflection of a light signal that is emitted by 62a and detected by 62b. However, that simple system cannot easily distinguish between a whole blood sample and some other liquid (e.g., blood serum) placed in the sample port in error or, even, an object (e.g., a finger) that can approach sample port 42 and cause the system to erroneously conclude that a proper sample has been applied. To avoid this type of error, another embodiment measures diffuse reflection from the sample port. This embodiment appears in Fig. 7A, which shows detector 62b positioned normal to the plane of strip 40. With the arrangement shown in Fig. 7A, if a whole blood sample has been applied to sample port 42, the signal detected by 62b increases abruptly, because of scattering in the blood sample, then decreases, because of rouleaux formation. The detector system 62 is thus programmed to require that type of signal before causing gimbaled bladder actuator 68 to release bladder 44. The delay of several seconds in releasing bladder 44 does not substantially affect the readings described below.

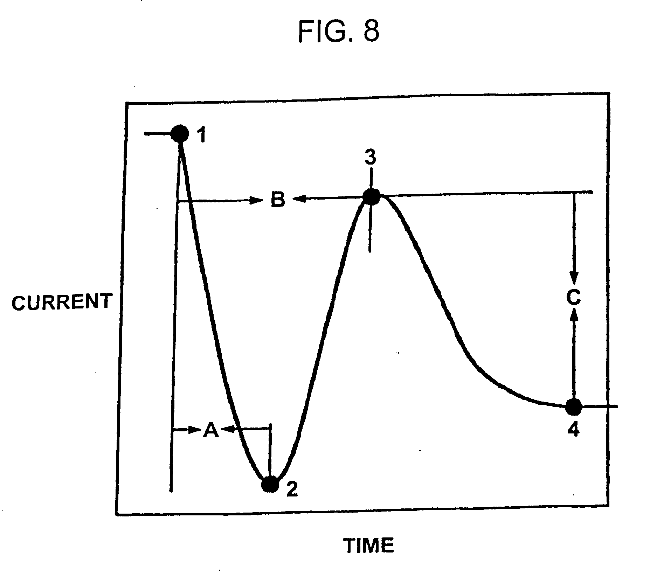

Fig. 8 depicts a typical "clot signature" curve in which the current from detector 44b is plotted as a function of time. Blood is first detected in the measurement area by detector 44b at time 1. In the time interval A, between points 1 and 2, the blood fills the measurement area. The reduction in current during that time interval is due to light scattered by red cells and is thus an approximate measure of the hematocrit. At point 2, sample has filled the measurement area and is at rest, its movement having been stopped by the stop junction. The red cells begin to stack up like coins (rouleaux formation). The rouleaux effect allows increasing light transmission through the sample (and less scattering) in the time interval between points 2 and 3. At point 3, clot formation ends rouleaux formation and transmission through the sample reaches a maximum. The PT time can be calculated from the interval B between points 1 and 3 or between 2 and 3. Thereafter, blood changes state from liquid to a semi-solid gel, with a corresponding reduction in light transmission. The reduction in current C between the maximum 3 and endpoint 4 correlates with fibrinogen in the sample.

### KITS

Also provided by the subject invention are kits for use in practicing the subject methods. The kits of the subject invention include at least one subject test strip compression elements, oftentimes a plurality of test strip compression elements, where compression elements may be the same or different. The kits may also include a reusable or disposable meter that may be used with subject test strip compression elements of the kit or from other kits. Certain kits may also include various types of test strips, e.g., for the same or different assays or the same assay, but configured differently such as including various numbers of measurement channels and the like. Finally, the kits may further include instructions for using the subject devices for determining the concentration of at least one analyte in a physiological sample. The instructions may be printed on a substrate, such as paper or plastic, *etc.* As such, the instructions may be present in the kits as a package insert, in the labeling of the container of the kit or components thereof (*i.e.,* associated with the packaging or sub-packaging) *etc.* In other embodiments, the instructions are present as an electronic storage data file present on a suitable computer readable storage medium, *e.g.,* CD-ROM, diskette, *etc.*

It is evident from the above description and discussion that the above-described invention provides an element which creates a substantially intimate, evenly distributed contact between a test strip an/or the measurement areas of a test strip, and a heater of a meter into which the test strip is inserted. The above-described invention provides a number of advantages, including the elimination of a source of error in analytical assays using such test strips. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

## Claims

1. A test strip compression element for use with a meter, said test strip compression element being configured to create a substantially intimate contact between a test strip and a heating element of said meter.

2. The test strip compression element of claim 1, wherein said compression element comprises at least one protrusion.

3. The test strip compression element of claim 2, wherein said at least one protrusion comprises a plurality of protrusions.

4. The test strip compression element of claim 2 or claim 3, wherein said at least one protrusion is configured to be positioned substantially adjacent to a measurement area of said test strip.

5. The test strip compression element of claim 1, further comprising one or more apertures.

6. The test strip compression element of claim 5, wherein at least one protrusion is positioned substantially adjacent said one or more apertures.

7. The test strip compression element of claim 1, wherein said compression element is spring-loaded.

8. The test strip compression element of claim 1, wherein said test strip compression element is configured to apply a force on said test strip to accomplish said substantially intimate, evenly distributed contact.

9. The test strip compression element of claim 8, wherein said applied force ranges in magnitude from 0.2 lbs. to 3 lbs.

10. A test strip meter, wherein said meter comprises a test strip compression element of any one of claims 1 to 9.

11. A method of creating a substantially intimate, evenly distributed contact between a tests trip and a heater of a meter, said method comprising:
applying a force to said test strip to create said substantially intimate, evenly distributed contact with a heater of said meter.
